# EUROPEAN PATENT SPECIFICATION

(11) **EP 1 859 827 B1**
(45) Date of publication and mention of the grant of the patent: **20.02.2013**
(21) Application number: 05800390.6
(22) Date of filing: 31.10.2005
(51) Int. Cl.: A61M 5/24, A61C 19/08, A61M 5/20

(54) **CHEMICAL-LIQUID INJECTION SPEED CONTROL METHOD AND DEVICE FOR CARTRIDGE-TYPE ELECTRIC INJECTOR FOR DENTAL APPLICATION**
VERFAHREN ZUR KONTROLLE DER INJEKTIONSGESCHWINDIGKEIT EINER CHEMISCHEN FLÜSSIGKEIT FÜR EIN ELEKTRISCHES INJEKTIONSGERÄT VOM KARTUSCHENTYP FÜR DENTALANWENDUNGEN
PROCÉDÉ DE CONTRÔLE DE VITESSE D'INJECTION DE LIQUIDE CHIMIQUE ET DISPOSITIF POUR SERINGUE ÉLECTRIQUE DE TYPE CARTOUCHE POUR APPLICATION DENTAIRE

(30) Priority: 24.02.2005 JP 2005049233
(43) Date of publication of application: 28.11.2007
(73) Proprietor: Showa Yakuhin Kako Co., Ltd, Tokyo 104-0031 (JP)
(72) Inventor: TANAKA, Fumio, Chuo-ku, Tokyo 104-0031 (JP); KAWASAKI, Yoshihiko, Chuo-ku, Tokyo 104-0031 (JP); HARAGUCHI, Mitsuhiro, Chuo-ku, Tokyo 104-0031 (JP); KITAGAWA, Ikuo, Matsudo-shi, Chiba 270-2223 (JP); KATO, Yoshinori, Matsudo-shi, Chiba 270-2223 (JP); HAYASHI, Renji, Matsudo-shi, Chiba 270-2223 (JP)
(74) Representative: Frost, Alex John
(86) International application number: PCT/JP2005/019989
(87) International publication number: WO 2006/090509

(56) References cited:
- JP-A- 11 500 038
- JP-A- 2003 533 287
- JP-A- 2004 130 005
- US-A- 5 180 371
- US-A- 5 807 334
- US-A1- 2002 077 601

## Description

### Background of the Invention:

### Field of the Invention:

The present invention relates to a cartridge type motor-driven injection syringe for dental use, and particularly to an apparatus for controlling the injection rate of a drug solution such as an anesthetic solution in a motor-driven injection syringe.

### Prior Art:

The local anesthesia is indispensable for removal of gingiva, preparation of cavities, extraction of tooth and the like. Nerves to be anaesthetized run in the jaw bone or tooth, and therefore, usually the anesthetic solution is not applied directly to the nerves. In almost all cases the injection needle is inserted in the soft tissue, periodontal ligament, or near the bone close to a selected nerve, and then the anesthetic solution is locally injected, allowing the anesthetic solution to spread all over the local area, thereby making the anesthetic solution work on the nerve, which is called "Infiltration Anesthesia".

Anesthetic-filled cartridges of different sizes (1.0 ml or 1.8 ml) are commercially available. In use such a cartridge is attached to a manually operable injection syringe, and a double-pointed injection needle is fixed to the injection syringe with one pointed end penetrated a rubber disc, by which the end of the cartridge is hermetically sealed. The cartridge has a plunger rubber movably fitted in the rear end of the cartridge for pushing the anesthetic solution out of the cartridge through the injection needle. A plunger rod of the injection syringe is operated with fingers to push the plunger rubber, thus applying a pressure to the anesthetic solution for discharging out of the cartridge. As may be realized, such intraligamentary injection requires application of a relatively high pressure to the injection syringe. In view of this a variety of motor-driven injection syringes for dental use have been developed as a substitute for the manually operable type of injection syringe, and have been commercially available.

The motor-driven injection syringe is basically equipped with a final reduction gear to reduce the speed of revolution of the electric motor, and a rack and pinion to convert the so reduced revolution into the linear movement of the plunger rod. To meet the demand for controlling the injection rate of the anesthetic solution in terms of the linear speed of the plunger rod (the injection rate being defined as a length of time involved for injecting the anesthetic solution of 1 ml) the present applicant has developed and marketed motor-driven injection syringes, which are so designed that dentists may choose among three different injection rates, that is, Low (200 secs/1 ml), Medium (100 sees/1 ml) and High (60 secs/1 ml) injection rates. The speed of revolution of the electric motor, therefore, is changed three stepwise, so that the plunger rod may travel at any one speed selected among Low (9.5 mm/min.), Medium (14.5 mm/min.) and High (23.0 mm/min.) speeds.

In dental local anesthesia patients feel pain twice, that is, at the time of needle insertion into the oral tissue and at the time of the flow of the anesthetic solution into the oral tissue. Nowadays injection needles have been improved such that the pain which patients feel in injection significantly lessens. Specifically, injection needles are reduced in outer diameter and thickness, and increased in inner diameter. Incidentally it is realized that the pain which patients feel at the beginning of injection lessens if the anesthetic solution is injected at a reduced flow rate. In view of this the motor-driven injection syringe is set at such a decreased speed that the pain at the beginning of the injection may noticeably lessen. The length of time involved for the injection can be shortened by setting the injection rate at the "Medium" or "High" although the problem of initial pain remains unsolved.

With a view to reaching a compromise some motor-driven injection syringes are equipped with automatic control means, which is programmed as for example follows: the injection rate gradually rises from zero for a predetermined length of time (3 seconds for low speed; 7 seconds for medium speed; and 10 seconds for high speed), and then, the injection rate is automatically changed for the Low rate (for example, 180 seconds/1 ml), the Medium rate (for example, 97 seconds/1 ml) or the High rate (for example, 70 seconds/1 ml) set by the dentist, and is kept at the set value for the remaining length of time. This effectively prevents patients against the initial pain. The programming of injecting operation, however, does not allow dentists to arbitrarily adjust the initial injection period prior to the switching to the Low , Medium or High rate.

### Summary of the Invention:

One object of the present invention is to provide an apparatus for controlling the injection rate of an anaesthetic solution in a cartridge type motor-driven injection syringe for dental use wherein the initial injection begins with the "Low" injection rate, and the consequent injection is made by switching from "Low" injection rate to the "Medium" or "High" injection rate at an arbitrarily selected time.

Another object of the present invention is to provide an apparatus for controlling the injection rate of an anaesthetic solution in a cartridge type motor-driven injection syringe for dental use wherein that the "low" injection rate for the initial injection can also be maintained for the whole period of injection.

The present invention provides an apparatus as defined in claim 1

Other objects and advantages of the present invention will be understood from the following description of one preferred embodiment of the present invention as shown in the drawings.

### Brief Description of the Drawings:

Fig.1 is a perspective view of a cartridge type of motor-driven injection syringe for dental use according to one embodiment of the present invention;
Fig.2 is a longitudinal section of the cartridge type of motor-driven injection syringe for dental use:

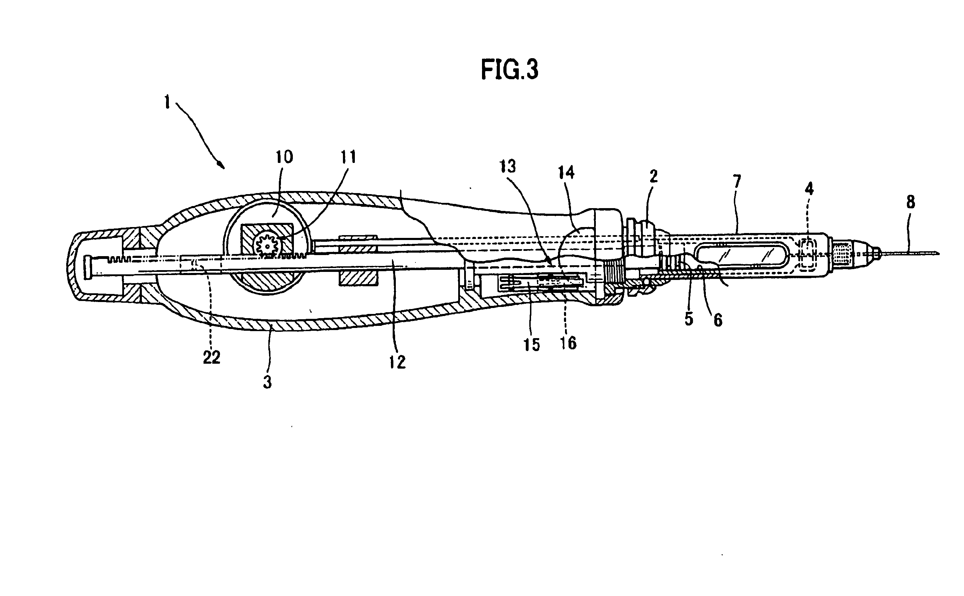

Fig.3 is a plane view of the motor-driven injection syringe of Fig.2, partially broken to show inside; and
Fig.4 is a block diagram showing the circuits for controlling the anaesthetic injection rate in the motor-driven injection syringe.

### Description of the Preferred Embodiment:

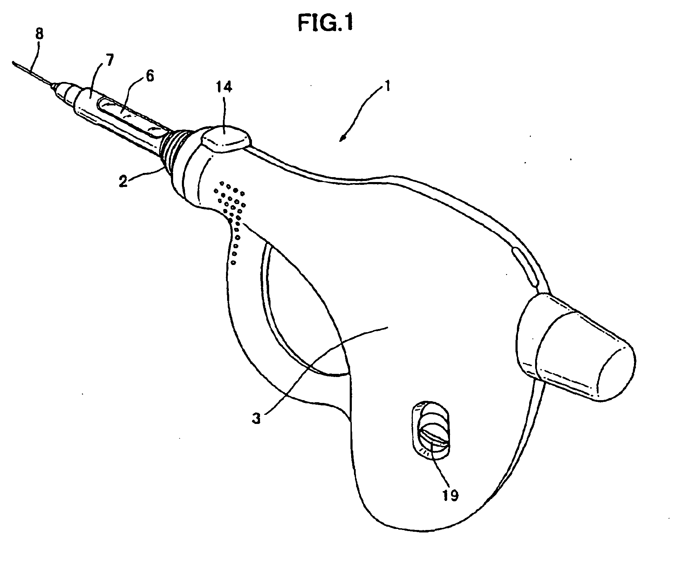

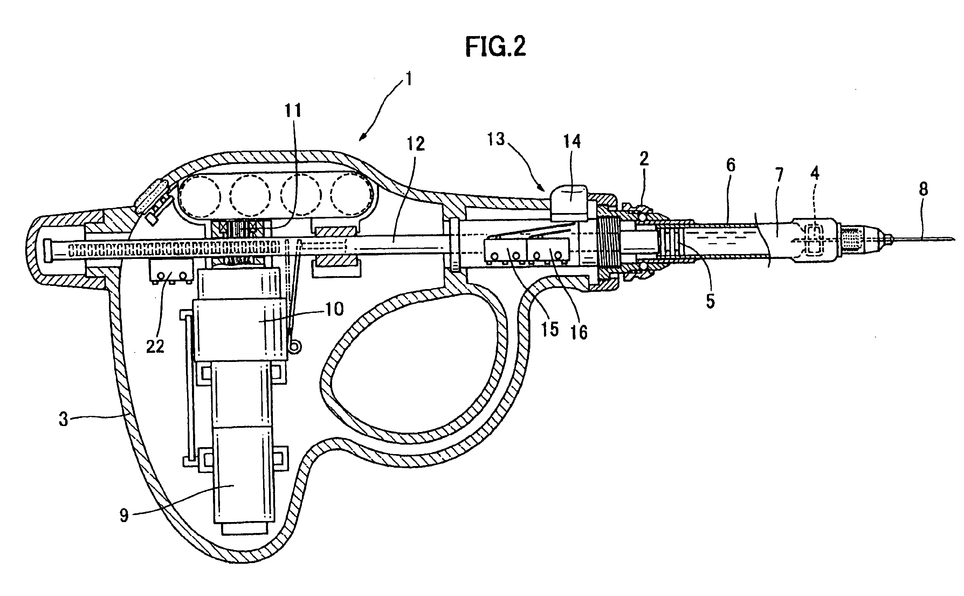

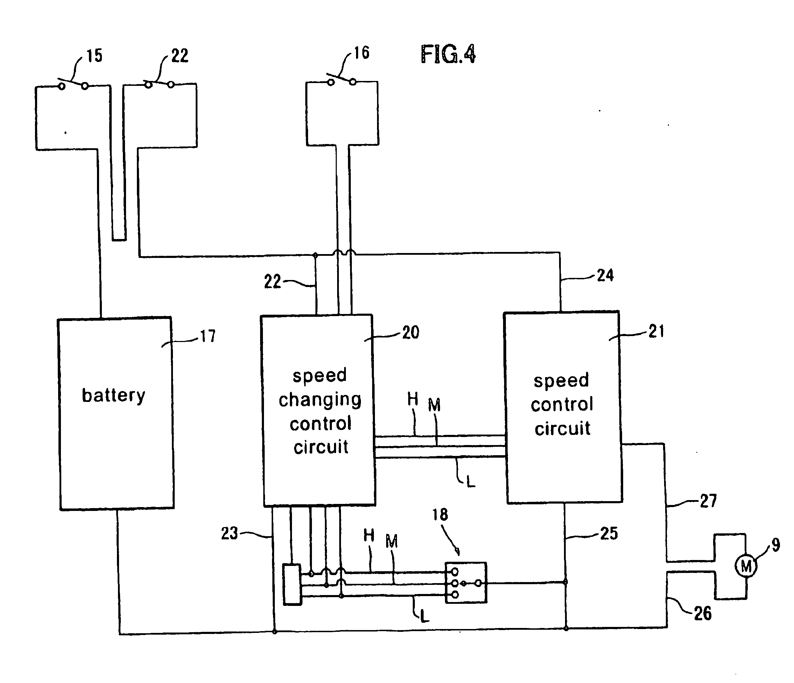

Fig.1 shows a cartridge type motor driven injection syringe 1 for dental use according to one embodiment of the present invention. As can be seen from Figs. 1 and 2, the motor-driven injection syringe 1 comprises a housing 3 with a nose 2, a cartridge holder 7 to accommodate an anaesthetic solution-filled cartridge 6, which has a rubber disc 4 and a plunger rubber 5 fitted therein, a double-pointed injection needle 8 detachably attached to the end of the cartridge holder 7, penetrating the rubber disc 4; a plunger rod 12 adapted to be linearly displaced by a rack and pinion 11 operatively connected through a final reduction gear 10 to an electric motor 9, thereby ejecting the anaesthetic solution from the cartridge 6 through the injection needle 8, a starter switch 13 for starting the electric motor 9, and a button 14 for operating the starter switch 13. The starter switch 13 comprises two sequential switches 15 and 16 responsive to first and second depressions for sequential operation, as described below with reference to a block diagram of Fig.4.

In the block diagram, an electric power supply or battery is indicated by 17. There is provided a speed selector switch 18 permiting a dentist to make a selection among the "Low Speed Circuit", "Medium Speed Circuit" and "High Speed Circuit" for rotating the electric motor 9 accordingly. The speed selector switch 18 has a knob slide 19 accessible from the outside of the cartridge type motor-driven injection syringe. A speed changing circuit 20 and a speed control circuit 21 are connected to each other by wires L, M and H in a central process unit. When the plunger rod 12 travels the forward stroke, a limit switch 22 opens the circuit to make the electric motor 9 stop.

When the first depression makes the switch 15 turn on, the speed changing circuit 20 is supplied with electricity from the battery 17 via the wires 22 and 23, and likewise the speed control circuit 21 is supplied with electricity from the battery 17 via the leads 24 and 25. The control circuit 20 sends a "Low Speed" signal to the speed control circuit 21 via the wire L, thereby making the electric motor 9 which is connected through wires 26,27 to the power supply 17 run at the "Low Speed". The speed selector switch 18 makes a selection among the "Low Speed Circuit", "Medium Speed Circuit" and "High Speed Circuit" by moving the knob slide 19. Subsequently, the second depression makes the switch 16 turn on. Then, the speed changing circuit 20 sends the "Low Speed", "Medium Speed" or "High Speed" signal to the speed control circuit 21 to make the electric motor 9 run at the selected speed of revolution. In any event, the speed of revolution of the electric motor 9 is reduced by the final reduction gear 10, and the plunger rod 12 is linearly moved by the rack and pinion at the reduced speed to push the plunger rubber 5, thereby ejecting the anaesthetic solution from the cartridge 6 through the injection needle 8.

In use, the knob slide 19 is moved such that the speed selector switch 18 select the "Medium Speed" or the "High Speed". Then, the first depression of the switch 13 causes the injection of the anaesthetic solution at the reduced injection rate. When it is confirmed that the patient is anethetized in an injection area, the dentist gives the second push to the switch 13 to inject the anaesthetic solution at the medium or high injection rate. In consideration of the patient's condition the dentist can adjust the length of time involved for the low injection rate. Thus, the dentist can shorten or lengthen the length of time involved for the anaesthetic injection as desired.

### List of Preference Numbers:

1 cartridge type motor-driven dental injection syringe.
2 nose
3 housing
4 rubber disc
5 plunger rubber
6 cartridge
7 cartridge holder
8 double-pointed injection needle
9 electric motor
10 final reduction gear
11 rack and pinion
12 plunger rod
13 two-push operable type of switch
14 button
15 switch
16 switch
17 battery
18 speed selector switch
19 knob slide
20 speed changing control circuit
21 speed control circuit
22 limit switch

## Claims

1. An apparatus for controlling the injection rate of an anaesthetic solution comprising a cartridge type motor-driven injection syringe for dental use, the motor-driven injection syringe comprising:
a housing (3) with a nose(2),
a cartridge holder (7) to accommodate an anaesthetic solution-filled cartridge (6) having a rubber disc (4) and a plunger rubber (5) fitted therein, the cartridge holder (7) being detachably attached to the nose (2);
a double-pointed injection needle (8) detachably attached to the end of the cartridge holder (7), for penetrating the rubber disc (4);
a plunger rod (12) adapted to be linearly displaced by a rack and pinion (11) operatively connected through a final reduction gear (10) to an electric motor (9), for ejecting the anaesthetic solution from the cartridge (6) through the injection needle (8);
a speed selector switch (18) actuatable for making a desired selection of the running speed of the electric motor (9) among a low speed, a medium speed and a high speed of the electric motor (9) by connection to a low speed circuit, medium speed circuit or a high speed circuit;
**characterized by** further comprising
a starter switch (13) for starting the electric motor (9), comprising two switches (15,16) responsive to first and second depressions for sequential operation; and
a speed changing circuit (20) and a speed control circuit (21) responsive to the first depression the starter switch (13) to turn on the electric motor (9) at the low speed, and responsive to the second depression of the starter switch (8) to make the electric motor (9) run at the desired running speed selected among the low speed, medium speed or high speed, dependent on connection to the low speed circuit medium speed circuit or high speed circuit.

## Patentansprüche

1. Vorrichtung zur Steuerung der Injektionsgeschwindigkeit einer anästhesierenden Lösung, die eine motorgetriebene Injektionsspritze vom Kartuschentyp zur dentalen Verwendung aufweist, wobei die motorgetriebene Injektionsspritze aufweist:
ein Gehäuse (3) mit einem Vorderteil (2),
eine Kartuschenhalterung (7) zur Aufnahme einer mit einer anästhesierenden Lösung gefüllten Kartusche (6), die eine Gummischeibe (4) und einen darin eingebauten Kolbengummi (5) aufweist, wobei die Kartuschenhalterung (7) lösbar mit dem Vorderteil (2) verbunden ist,
eine zweispitzige Injektionsnadel (8), die zum Durchdringen der Gummischeibe (4) lösbar an dem Ende der Kartuschenhalterung (7) angebracht ist;
einen Kolbenstab (12), der zum Ausstoßen der anästhesierenden Lösung aus der Kartusche (6) in die Injektionsnadel (8) für eine lineare Verschiebung durch eine Zahnstange (11) angepasst ist, die über ein Enduntersetzungsgetriebe (10) operativ mit einem Elektromotor (9) verbunden ist;
einen Geschwindigkeitswahlschalter (18), der für eine gewünschte Auswahl der Laufgeschwindigkeit des Elektromotors (9) unter einer niedrigen Geschwindigkeit, einer mittleren Geschwindigkeit und einer hohen Geschwindigkeit des Elektromotors (9) durch Verbindung mit einem Schaltkreis für niedrige Geschwindigkeit, einem Schaltkreis für mittlere Geschwindigkeit oder einem Schaltkreis für hohe Geschwindigkeit betätigbar ist;
wobei sie **dadurch gekennzeichnet ist, dass** sie ferner aufweist:
einen Startschalter (13) zum Starten des Elektromotors (9), der zwei Schalter (15, 16) umfasst, die für den Folgebetrieb auf ein erstes und ein zweites Drücken ansprechen; und
einen Schaltkreis zur Änderung der Geschwindigkeit (20) und einen Schaltkreis zur Steuerung der Geschwindigkeit (21), der auf das erste Drücken des Startschalters (13) anspricht, um den Elektromotor (9) auf die niedrige Geschwindigkeit zu bringen; und auf das zweite Drücken des Startschalters (13) anspricht, um den Elektromotor (9) in Abhängigkeit vom Anschluss an den Schaltkreis für die niedrige Geschwindigkeit, den Schaltkreis für die mittlere Geschwindigkeit oder den Schaltkreis für die hohe Geschwindigkeit auf der gewünschten Laufgeschwindigkeit, die unter der niedrigen Geschwindigkeit, der mittleren Geschwindigkeit oder der hohen Geschwindigkeit ausgewählt ist, zu betreiben;

## Revendications

1. Appareil pour commander la vitesse d'injection d'une solution anesthésique comprenant une seringue d'injection actionnée par moteur du type cartouche destinée à un usage dentaire, la seringue d'injection actionnée par moteur comprenant :
un logement (3) pourvu d'un nez (2),
un support de cartouche (7) destiné à recevoir une cartouche remplie de solution anesthésique (6) comportant un disque de caoutchouc (4) et un caoutchouc de piston (5) emboîté en son sein, le support de cartouche (7) étant fixé, de manière amovible, au nez (2) ;
une aiguille d'injection à deux pointes (8) fixée, de manière amovible, à l'extrémité du support de cartouche (7), pour pénétrer dans le disque de caoutchouc (4);
une tige de piston (12) apte à être déplacée linéairement par un mécanisme à crémaillère (11) fonctionnellement relié, par le biais d'un engrenage réducteur final (10), à un moteur électrique (9), pour éjecter la solution anesthésique de la cartouche (6) par l'aiguille d'injection (8) ;
un commutateur de sélection de vitesse (18) pouvant être actionné pour réaliser une sélection souhaitée de la vitesse de fonctionnement du moteur électrique (9) entre une faible vitesse, une vitesse moyenne et une vitesse élevée du moteur électrique (9) par connexion à un circuit de faible vitesse, un circuit de vitesse moyenne ou un circuit de vitesse élevée ;
**caractérisé en ce qu'**il comprend, en outre :
un commutateur de démarrage (13) pour démarrer le moteur électrique (9), comprenant deux commutateurs (15, 16) réagissant à des premier et second enfoncements pour un fonctionnement séquentiel ; et
un circuit de changement de vitesse (20) et un circuit de commande de vitesse (21) réagissant au premier enfoncement du commutateur de démarrage (13) pour mettre en marche le moteur électrique (9) à la faible vitesse, et réagissant au second enfoncement du commutateur de démarrage (13) pour faire fonctionner le moteur électrique (9) à la vitesse de fonctionnement sélectionnée souhaitée parmi la faible vitesse, la vitesse moyenne ou la vitesse élevée, en fonction de la connexion au circuit de faible vitesse, circuit de vitesse moyenne ou circuit de vitesse élevée.
